# EUROPÄISCHE PATENTSCHRIFT

(11) **EP 2 338 420 B1**
(45) Veröffentlichungstag und Bekanntmachung des Hinweises auf die Patenterteilung: **29.05.2013**
(21) Anmeldenummer: 10016014.2
(22) Anmeldetag: 23.12.2010
(51) Int. Cl.: A61B 17/00

(54) **Medizinisches Implantat zum Verschliessen von Gefässöffnungen**
Medical implant for closing off veins
Implant médical destiné à la fermeture d'ouvertures de récipients

(30) Priorität: 23.12.2009 DE 202009017418 U; 04.06.2010 EP 10005799
(43) Veröffentlichungstag der Anmeldung: 29.06.2011
(73) Patentinhaber: Bentley InnoMed Gmbh, 72379 Hechingen (DE)
(72) Erfinder: Obradovic, Milisav, 79539 Lörrach (DE); Eisold, Gerd, 72415 Groselfingen (DE)
(74) Vertreter: Thiel, Christian

(56) Entgegenhaltungen:
- WO-A1-2007/115117
- US-A1- 2006 116 710
- US-A1- 2007 083 227
- US-A1- 2008 249 562

## Beschreibung

Die Erfindung betrifft ein medizinisches Implantat zum Verschließen von Gefäßöffnungen und/oder Shunt-Verbindungen zwischen Körpergefäßen, das in einem kontrahierten Zustand minimal-invasiv positionierbar und in einen expandierten Zustand überführbar ist, indem es zwei voneinander beabstandete, im Durchmesser erweiterte Dichtelemente aufweist, die die Gefäßöffnung oder Shunt-Verbindung auf beiden Seiten abdichten, wobei das Implantat aus einem rohrförmigen Körper durch Einbringen von Wandöffnungen mindestens im Bereich der im expandierten Zustand einen erweiterten Durchmesser aufweisenden Dichtelement hergestellt ist.

Solche auch als Okkluder bezeichnete Implantate sind bereits bekannt. So beschreibt beispielsweise die EP 0 808 138 B1 einen Okkluder, der aus einem Metallgewebe aus miteinander verflochtenen Metalllitzen gefertigt ist. Der Okkluder wird mit Hilfe eines Katheters an die gewünschte Stelle im vaskulären System eines Patienten platziert und in den expandierten Zustand gebracht. Mit Hilfe des Okkluders lassen sich Gefäße eines Patienten verschließen, beispielsweise septale Defekte, wie etwa Defekte des Vorhofseptums. Auch ein persistierender Ductus (PDA) oder ein Ventrikelseptumdefekt können mit Okkludern behandelt werden. Ein weiteres, ähnliches Okklusionsinstrument ist aus der DE 10 2005 053 958 Al bekannt. Auch dieses Okklusionsinstrument besteht aus einem Metallgewebe mit zwei schirmartigen Dichtelementen, wobei im implantierten Zustand eines der Dichtelemente auf der einen Seite der Gefäßöffnung und das andere Dichtelement auf der gegenüberliegenden Seite der Gefäßöffnung an der Gefäßwand anliegt und die zu schließende Gefäßöffnung abdichtet. Die US 2008/0249562 A1 offenbart den Gegenstand des Oberbegriffs des Anspruchs 1.

Die bekannten Okkluder haben den Nachteil, dass ihre Fertigung aus dünnen Metalldrähten oder Polymerfasern relativ aufwändig ist.

Der vorliegenden Erfindung liegt daher die Aufgabe zugrunde, ein Okklusionsimplantat zu schaffen, das einfach in der Herstellung und in der Handhabung ist.

Die Aufgabe wird mit einem medizinischen Implantat zum Verschließen von Gefäßöffnungen und/oder Shunt-Verbindungen zwischen Körpergefäßen gelöst, das in einem kontrahierten Zustand minimal-invasiv positionierbar und in einen expandierten Zustand überführbar ist, indem es zwei voneinander beabstandete, im Durchmesser erweiterte Dichtelemente aufweist, mit denen die Gefäßöffnung oder Shunt-Verbindung auf beiden Seiten abdichtbar ist, wobei das Implantat aus einem rohrförmigen Körper durch Einbringen von Wandöffnungen mindestens im Bereich der im expandierten Zustand einen erweiterten Durchmesser aufweisenden Dichtelemente hergestellt ist und wobei in expandiertem Zustand die Stege eines jeden Dichtelements im Wesentlichen rechtwinklig zur Längsachse des Implantats verlaufen und im Bereich jedes Dichtelements radiale Schlaufen ausbilden, die zur Längsachse des Implantats hin offen sind, wobei die Schlaufen eines jeden Dichtelements jeweils im Wesentlichen in einer Ebene liegen und die Schlaufen der beiden Dichtelemente im zentralen Bereich über im Wesentlichen gerade und parallel zueinander verlaufende Stege miteinander verbunden sind.

Die im Wesentlichen rechtwinklig zur Längsachse des Implantats verlaufenden Stege im Bereich der Dichtelemente erlauben es, das Implantat mit einer sehr geringen Längenausdehnung herzustellen. Eine weitere Längenverkürzung ergibt sich daraus, dass die Stege der Dichtelemente radiale Schlaufen ausbilden, die in jedem Dichtelement für sich im Wesentlichen in einer Ebene liegen. Auch dieses Merkmal ist Wesentlich für eine kurze Ausgestaltung des Implantats.

Die Anordnung der Stege in zur Längsachse des Implantats offenen Schlaufen führt zu einer schirmförmigen oder blütenähnlichen Ausgestaltung der Dichtelemente, die sich gegebenenfalls leicht mit einer Membran bespannen lassen. Die Schlaufen erlauben eine gewisse Federwirkung, weshalb sich das Implantat sehr einfach an die zu verschließende Öffnung, beispielsweise im Septum des Herzens, anpassen kann. Auch unregelmäßige Öffnungen sind damit einfach zu verschließen.

Schließlich erlaubt der im Wesentlichen gerade Verlauf der Stege im zentralen Bereich, die die Schlaufen der beiden Schirme miteinander verbinden, eine optimale Anpassung an die Dicke des Septums. Im Verlauf dieser Stege kommt es häufig zu Anpassungsschwierigkeiten, insbesondere dann, wenn die Stege im zentralen Bereich gekrümmt und der Septumverlauf nicht gleichmäßig ist. Quetschungen bzw. Schädigungen der Septumoberfläche sind möglich, insbesondere unter dem Gesichtspunkt, dass das Herz ein sich ständig bewegender Muskel ist.

Der gerade und parallele Verlauf der Stege im zentralen Bereich, die die Schlaufen der beiden Dichtelemente miteinander verbinden, erlaubt zudem die variable Herstellung der Implantate, d.h. die individuelle Anpassung an die physiologischen Verhältnisse des jeweiligen Patienten.

Die erfindungsgemäßen Implantate werden also nicht aus Geweben oder Netzen hergestellt, die zuvor in relativ aufwändigen Verfahren aus dünnen Fäden oder Drähten gefertigt werden. Stattdessen wird das Implantat aus einem massiven rohrförmigen Körper gefertigt, der zumindest an den Stellen, die im expandierten Zustand die Dichtelemente bilden, mit Wandöffnungen versehen wird. Durch das Einbringen der Wandöffnungen ist es möglich, die Dichtelemente mit den erweiterten Durchmessern herzustellen, beispielsweise indem der rohrförmige Körper gestaucht wird. Die Wandöffnungen lassen sich vorzugsweise durch Laserschneiden in den rohrförmigen Körper einbringen. Dieses Verfahren ist hochpräzise und relativ preisgünstig. Der gegenseitige Abstand der Wandöffnungen kann auch derart gewählt werden, dass die dazwischen stehen bleibenden Wandstege des rohrförmigen Körpers dicker sind als die Fäden oder Litzen der bekannten Okkluder. Dadurch lassen sich erfindungsgemäße Implantate mit einer höheren Formstabilität als die bekannten Okkluder herstellen.

Die Wandöffnungen sind dabei schlitzförmig ausgebildet. Diese Form der Wandöffnungen ermöglicht eine leichte Verformbarkeit des rohrförmigen Körpers, sodass die im expandierten Zustand einen erweiterten Durchmesser aufweisenden Dichtelemente einfach herstellbar sind. Weitere Vorteile ergeben sich, wenn mehrere, parallel zueinander ausgerichtete Wandöffnungen vorgesehen sind. Auch diese Maßnahme erleichtert die Verformbarkeit des Implantats. Der gegenseitige Abstand der Wandöffnungen und ihre Verteilung über den Umfang des rohrförmigen Körpers kann entsprechend den gewünschten Eigenschaften des Implantats gewählt werden. Die Konfiguration der Wandöffnungen bestimmt die Eigenschaften der Dichtelemente.

Bei einer bevorzugten Variante können sich die Wandöffnungen im Wesentlichen parallel zur Längsachse des Implantats erstrecken. Dadurch sind sie einfach in den rohrförmigen Körper einzubringen und gewährleisten außerdem ein leichtes Überführen des Implantats in den expandierten Zustand. Die Wandöffnungen können jedoch auch in einem spitzen Winkel zur Längsachse des Implantats verlaufen oder sich spiralförmig über den Umfang des Implantats erstrecken.

Weitere Vorteile ergeben sich, wenn das Implantat aus einer Formgedächtnislegierung, insbesondere aus Nitinol gefertigt ist. Bei der Verwendung solcher Materialien lässt sich der expandierte Zustand des Implantats fixieren. Nitinol weist zugleich federnde Eigenschaften auf, sodass das Implantat zum Einführen in den Körper des Patienten in den kontrahierten Zustand gebracht und beispielsweise mittels eines Katheters eingeführt werden kann. Am Zielort kann der Katheter zurückgezogen werden, wodurch das Implantat die abgespeicherte expandierte Form annimmt, indem sie in der Lage ist, die Gefäßöffnung oder die ShuntVerbindung abzudichten.

Alternativ zur Verwendung einer Formgedächtnislegierung kann auch ein Formgedächtnispolymer zur Herstellung des erfindungsgemäßen Implantats verwendet werden. Polymere haben ebenfalls hervorragende Formgedächtniseigenschaften und lassen sich noch einfacher verformen als Nitinol.

Zur Erzielung einer flüssigkeitsdichten Abdichtung von Gefäßöffnungen können in oder auf den Dichtelementen flüssigkeitsdichte Membranen angeordnet sein. Diese Membranen werden mit Hilfe der Dichtelemente beidseits der Gefäßöffnungen positioniert und in dieser Lage gehalten. Die Membranen können aus unterschiedlichen Materialien gefertigt sein. Urn eine hohe Körperverträglichkeit zu erzielen, ist ePTFE als Material für die Membranen bevorzugt. Solche Membranen können beispielsweise an Durchbrechungen der Stege im Bereich der Schlaufen und/oder im zentralen Bereich fest genäht werden.

Zur Herstellung des Implantats werden Wandöffnungen in den rohrförmigen Körper nicht nur in den Bereichen der Dichtelemente, sondern auch in einem zentralen Bereich zwischen den Dichtelementen eingebracht. Dadurch ist es möglich, durch Stauchen und/oder Verwinden dieses zentralen Bereichs den Durchmesser des Implantats und den gegenseitigen Abstand der Dichtelemente zu verändern und/oder den zentrale Bereich in seinem Querschnitt dem Querschnitt der Gefäßöffnung oder der Shunt-Verbindung anzupassen. Damit lässt sich eine gute Zentrierung des Implantats in der Gefäßöffnung oder Shunt-Verbindung erzielen. Gleichzeitig werden die Dichtelemente gegen die Gefäßwand in Anlage gebracht, das heißt eine gute Dichtwirkung des Implantats erreicht.

Dabei gehen die schlitzförmigen Wandöffnungen des zentralen Bereichs in die schlitzförmigen Wandöffnungen im Bereich der Dichtelemente über. Die Wandöffnungen werden somit als durchgehende, sich bis zu den Endbereichen des Rohres erstreckende Schlitze gefertigt. Der Verzicht auf einen nicht geschlitzten Rohrabschnitt zwischen dem zentralen Bereich und den Dichtelementbereichen führt außerdem zu einer leichteren Verformbarkeit des zentralen Bereichs. Er kann damit einfacher an unterschiedliche Größen und Formen von Gefäßöffnungen oder ShuntVerbindungen angepasst werden. Häufig weisen diese keinen runden, sondern einen ovalen oder schlitzförmigen Querschnitt auf. Aufgrund der flexiblen Gestaltung des zentralen Bereichs können jedoch mit einem einzigen Typ von Implantat eine ganze Reihe von Öffnungsgrößen und -formen abgedeckt werden. Die bisher erforderliche Bevorratung von Implantaten für unterschiedliche Durchmessergrößen der zu verschließenden Öffnungen im 1-Millimeter-Raster kann somit deutlich reduziert werden. Es können jetzt mit einem Implantat Durchmesserbereiche der Öffnungen von mehreren Millimetern versorgt werden.

Bei einer bevorzugten Ausgestaltung können die Wandöffnungen im zentralen Bereich parallel zur Längsachse des Implantats und im Bereich der beiden Dichtelemente spiralförmig verlaufen. Nach dem Überführen in den expandierten Zustand bilden in den Dichtelementbereichen die zwischen den Wandöffnungen verbleibenden Stege sich radial nach außen erstreckende Schlaufen. Der zentrale Bereich kann ebenfalls gestaucht werden, wodurch er eine bauchige Form annimmt. Durch Verdrehen um die Längsachse kann er außerdem in der Länge verkürzt werden. Der Umfang des zentralen Bereichs passt sich nach dem Positionieren des Implantats an der zu verschließenden Stelle dem Umfang der Gefäßöffnung oder der Shunt-Verbindung an.

Wie die bekannten Okkluder kann auch das erfindungsgemäße Implantat vorzugsweise mit Hilfe eines Katheters oder einer Wendel im Körper positionierbar sein. Zusätzlich kann das erfindungsgemäße Implantat durch einen Führungsdraht geführt und positioniert werden. Mit Hilfe des Katheters oder der Wendel kann das Implantat mit hoher Genauigkeit durch das vaskuläre System des Patienten bewegt und am gewünschten Einsatzort positioniert werden. Für die Befestigung des Katheters oder der Wendel kommen unterschiedliche Möglichkeiten in Betracht. Bei einer besonders einfachen Ausgestaltung kann der Katheter oder die Wendel mittels einer Schraubverbindung oder mittels eines Bajonettverschlusses mit einem der Enden des Implantats verbindbar sein.

Nach dem Einsetzen des Implantats soll dieses von Körpergewebe überwuchert werden. Dies lässt sich dadurch erleichtern, dass das gesamte Implantat eine biokompatible Beschichtung aufweisen kann.

Nachfolgend werden bevorzugte Ausführungsbeispiele eines erfindungsgemäßen Implantats anhand der Zeichnungen näher beschrieben.

Im Einzelnen zeigen:
- Fig. 1: eine perspektivische Ansicht eines rohrförmigen Körpers zur Herstellung eines erfindungsgemäßen Implantats;
- Fig. 2: eine perspektivische Ansicht des Implantats von Fig. 1 im expandierten Zustand;
- Fig. 3: eine Stirnansicht auf das Implantat von Fig. 1.

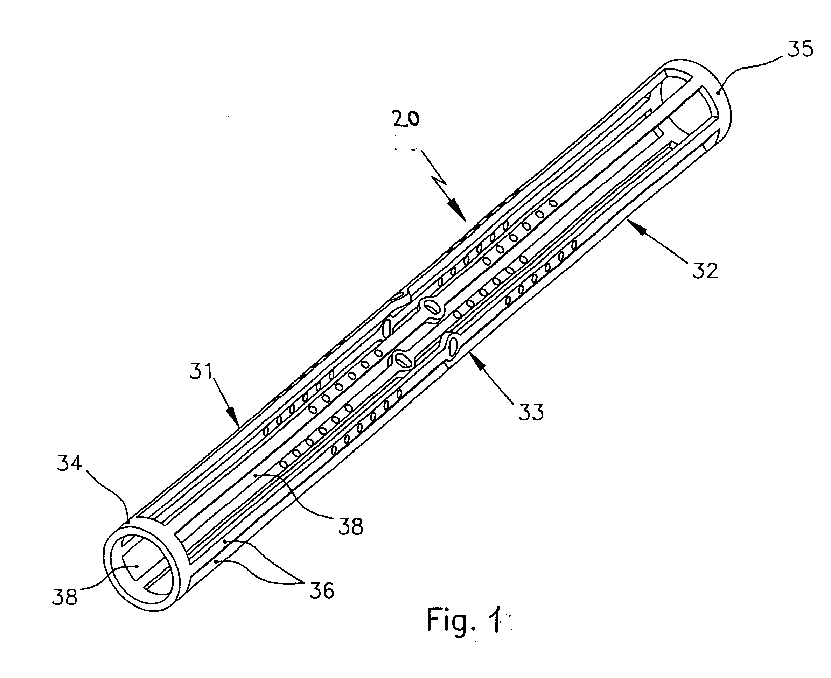

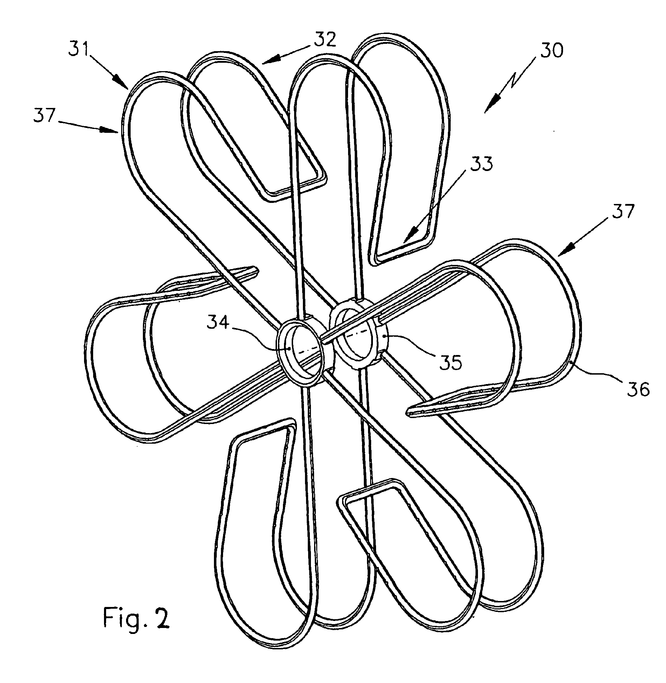

Fig. 1 zeigt einen rohrförmigen Körper 20 zur Herstellung eines Implantats 30, das sich über die beiden Dichtungsbereiche 31 und 32 und einen dazwischen liegenden zentralen Bereich 33 erstreckende Wandöffnungen 38 aufweist, die außerdem relativ breit sind, sodass dazwischen nur wenige Wandstege 36 verbleiben. Die Wandöffnungen 38 sind somit als durchgehende, sich bis zu den Endbereichen 34 und 35 des Rohres erstreckende Schlitze gefertigt. Der Verzicht auf nicht geschlitzte Rohrabschnitte zwischen dem zentralen Bereich 33 und den Dichtelementbereichen 31 und 32 führt zu einer leichten Verformbarkeit des zentralen Bereichs 33. Er lässt sich damit einfach an unterschiedliche Größen und Formen von Gefäßöffnungen oder Shunt-Verbindungen anpassen. Dies verdeutlicht Fig. 2, die das Implantat 30 im expandierten Zustand zeigt. Die Wandstege 36 bilden im Bereich der Dichtelemente 31 und 32 radial nach außen ragende Schlaufen 37. Im zentralen Bereich 33 verlaufen die Wandstege 36 parallel zueinander, sind radial jedoch nach außen gedrückt worden, sodass sie im Vergleich zu den Rohrenden 34, 35 einen deutlich größeren Durchmesser zwischen sich einschließen, im gezeigten Fall etwa den fünffachen Durchmesser.

Die radial nach außen ragenden Schlaufen 37 der Dichtelemente 31 und 32 verlaufen in etwa parallel und gleichsinnig. Die Schlaufen eines Dichtelements liegen dabei im Wesentlichen in einer Ebene. Schlaufen 37 verschiedener Dichtelemente sind im zentralen Bereich 33 durch gerade verlaufende Stege 36 miteinander verbunden. Der gleichsinnige Verlauf der Schlaufen der jeweiligen Dichtelemente und die Verbindung über Stege 36 im zentralen Bereich 33 ergibt eine gewisse Federwirkung, die eine einfache Anpassung der Dichtelemente und des Implantats an die zu verschließende Öffnung bewirkt.

In Fig. 1 ist noch die Durchbrechung einzelner Wandstege 36 im Bereich der Dichtelemente 31 und 32 und im zentralen Bereich 33 dargestellt, die das Annähen einer flüssigkeitsdichten Membran an die Dichtelemente ermöglichen.

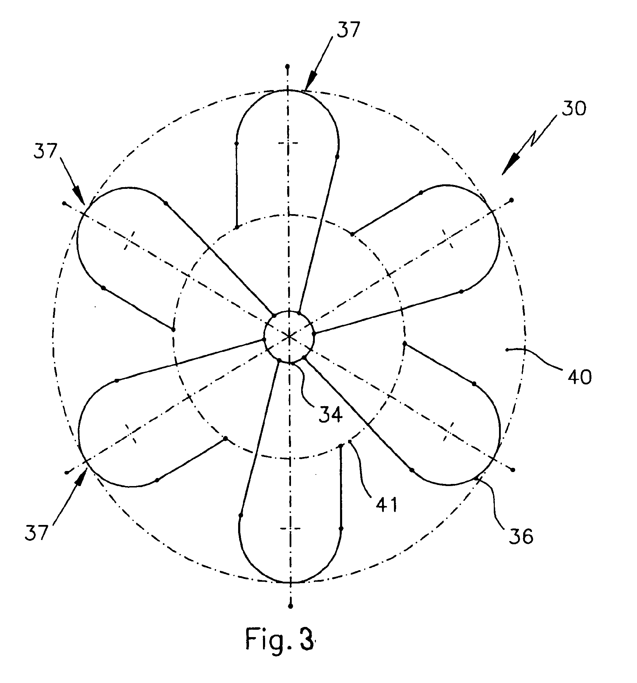

In Fig. 3 ist die Stirnansicht des Implantats 30 dargestellt, aus der auch die Anordnung einer strichpunktiert eingezeichneten Membran 40 im Dichtbereich 31 deutlich wird. Die Membran 40 aus einem flüssigkeitsdichten Material kann zwischen den beiden Hälften der radialen Schlaufen 37 aufgespannt und beispielsweise an den äußeren Bögen der Schlaufen 37 befestigt werden. Sie ist damit in der Lage, eine Gefäßöffnung, deren Durchmesser in etwa dem Durchmesser des Kreises 41 entspricht, sicher zu verschließen. Auf dem Kreis 41, der im allgemeinen etwa den 2- bis 15-fachen und insbesondere den 4- bis 10-fachen Durchmesser des Rohres 20 hat, liegen die Abschnitte der Wandstege 36 (Fig. 2) im zentralen Bereich 33 des Implantats 30, der nach dem Einsetzen des Implantats 30 durch die Gefäßöffnung hindurchragt. Auch im Dichtbereich 32 wird eine Membran 40 angeordnet, sodass die Gefäßöffnung von beiden Seiten her abgedichtet ist. Es versteht sich, dass die Implantate 10' und 30 auch in Gefäßöffnungen oder Shunt-Verbindungen eingesetzt werden können, die keinen kreisrunden, sondern einen eher ovalen oder schlitzförmigen Querschnitt aufweisen. Durch ein entsprechendes Verformen der Wandstege 36 im zentralen Bereich 33 können diese auch bei solchen Gefäßöffnungen in Anlage an den Rand der Öffnung gebracht werden und dadurch das Implantat 30 zentrieren.

Die Implantate können beispielsweise aus Nitinol oder einer anderen Metalllegierung mit Formengedächtnis oder aber auch aus einem Formgedächtnispolymer hergestellt sein. Die Membranen 40 im Bereich der Dichtelemente können vorzugsweise aus ePTFE gefertigt sein, da sich dieses Material durch eine sehr gute Körperverträglichkeit auszeichnet.

Die in den Figuren gezeigten Implantate 30 wurden durch Einbringen von schlitzförmigen Wandöffnungen 38 hergestellt, die parallel zur Längsachse des rohrförmigen Körpers 20 verlaufen. Dies ist jedoch nicht zwingend. Die Öffnungen 38 können mit der Längsachse des rohrförmigen Körpers 20 auch einen Winkel bilden, wodurch auch die Stege 36 Schlaufen bilden, deren Anfangs- und Endpunkte in tangentialer Richtung versetzt zueinander sind. Auch spiralförmig verlaufende Wandöffnungen oder andere Verläufe sind möglich.

## Patentansprüche

1. Medizinisches Implantat (30) zum Verschließen von Gefäßöffnungen und/oder Shunt-Verbindungen zwischen Körpergefäßen, das in einem kontrahierten Zustand minimal-invasiv positionierbar und in einen expandierten Zustand überführbar ist, indem es zwei voneinander beabstandete, im Durchmesser erweiterte Dichtelemente (31, 32) aufweist, die die Gefäßöffnung oder Shunt-Verbindung auf beiden Seiten abdichten, wobei das Implantat (30) aus einem rohrförmigen Körper (20) durch Einbringen von Wandöffnungen (38) unter Ausbildung von Stegen (36) im Bereich der im expandierten Zustand einen erweiterten Durchmesser aufweisenden Dichtelemente (31, 32) und im zentralen Bereich (33) zwischen den Dichtelementen (31, 32) hergestellt ist, **dadurch gekennzeichnet, dass** die Wandöffnungen (38) als durchgehende, sich zu den Endbereichen (34, 35) des rohrförmigen Körpers (20) erstreckende Schlitze gefertigt sind, und im expandierten Zustand die Stege (36) eines jeden Dichtelements (31,32) im Wesentlichen rechtwinklig zur Längsachse des Implantats (30) verlaufen und im Bereich jedes Dichtelements (31, 32) radiale Schlaufen (37) ausbilden, die zur Längsachse des Implantats (30) offen sind, wobei die Schlaufen (37) eines jeden Dichtelements (31, 32) im Wesentlichen in einer Ebene liegen und die Schlaufen (37) der beiden Dichtelemente (31, 32) im zentralen Bereich (33) über im Wesentlichen gerade und parallel zueinander verlaufende Stege (36) miteinander verbunden sind.

2. Implantat nach Anspruch 1, **dadurch gekennzeichnet, dass** mehrere, parallel zueinander ausgerichtete Wandöffnungen (38) vorgesehen sind.

3. Implantat nach einem der vorhergehenden Ansprüche, **dadurch gekennzeichnet, dass** sich die Wandöffnungen (38) im Wesentlichen parallel zur Längsachse des Implantats (30) erstrecken.

4. Implantat nach einem der Ansprüche 1 bis 2, **dadurch gekennzeichnet, dass** die Wandöffnungen (38) in einem spitzen Winkel zur Längsachse des Implantats (30) angeordnet sind.

5. Implantat nach einem der Ansprüche 1 bis 2, **dadurch gekennzeichnet, dass** sich die Wandöffnungen (38) spiralförmig über den Umfang des Implantats (30) erstrecken.

6. Implantat nach einem der vorhergehenden Ansprüche, **dadurch gekennzeichnet, dass** die die Schlaufen (37) der beiden Dichtelemente (31, 32) im zentralen Bereich (33) verbindenden Stege (36) in etwa auf dem Durchmesser eines Kreises (41) liegen, der gegenüber dem rohrförmigen Körper (20) einen erweiterten Durchmesser hat.

7. Implantat nach Anspruch 6, **dadurch gekennzeichnet, dass** der Durchmesser des Kreises (41) gegenüber dem Durchmesser des rohrförmigen Körpers (20) um einen Faktor von 2 bis 15, vorzugsweise 4 bis 10 erweitert ist.

8. Implantat nach einem der vorhergehenden Ansprüche, **dadurch gekennzeichnet, dass** die Stege (36) im Bereich der Schlaufen (37) und/oder im zentralen Bereich (33) Durchbrechungen zur Festlegung einer Membran (40) aufweisen.

9. Implantat nach einem der vorhergehenden Ansprüche, **dadurch gekennzeichnet, dass** es aus dem expandierten Zustand durch eine reversible elastische oder pseudoelastische Verformung in den kontrahierten Zustand überführbar ist.

10. Implantat nach einem der vorhergehenden Ansprüche, **dadurch gekennzeichnet, dass** es aus einer Formgedächtnislegierung, insbesondere aus Nitinol, oder aus einem Formgedächtnispolymer gefertigt ist.

11. Implantat nach einem der vorhergehenden Ansprüche, **dadurch gekennzeichnet, dass** in oder auf den Dichtelementen (31, 32) flüssigkeitsdichte Membranen (40) angeordnet sind.

12. Implantat nach Anspruch 11, **dadurch gekennzeichnet, dass** die Membranen (40) aus ePTFE gefertigt sind.

13. Implantat nach einem der Ansprüche 6 bis 12, **dadurch gekennzeichnet, dass** durch Stauchen und/oder Verwinden des zentralen Bereichs (33) die beiden Dichtelemente (31, 32) gegen die die Gefäßöffnung oder Shunt-Verbindung begrenzende Gefäßwand in Anlage bringbar sind und/oder der zentrale Bereich (33) in seinem Querschnitt dem Querschnitt der Gefäßöffnung oder der Shunt-Verbindung anpassbar ist.

14. Implantat nach einem der vorhergehenden Ansprüche, **dadurch gekennzeichnet, dass** es mit Hilfe eines Katheters oder einer Wendel im Körper positionierbar ist.

15. Implantat nach Anspruch 14, **dadurch gekennzeichnet, dass** der Katheter oder die Wendel an mindestens einem der Enden (34, 35) des Implantats (30) befestigbar ist.

16. Implantat nach Anspruch 15, **dadurch gekennzeichnet, dass** der Katheter oder die Wendel in eines der rohrförmigen Enden (34, 35) des Implantats einschraubbar oder mittels eines Bajonettverschlusses befestigbar ist.

## Claims

1. A medical implant for closing off vessel openings and/or shunt connections between bodily vessels, which is positionable in a minimally invasive manner in a contracted state and is convertible into an expanded state due to the fact that it has two sealing elements (31, 32) of increased diameter, which are spaced from one another and which seal the vessel opening or shunt connection on both sides, wherein the implant (30) is produced from a tubular body (20) by forming wall openings (38) whilst forming webs (36) in the region of the sealing elements (31, 32), which have an increased diameter in the expanded state, and in the central region (33) between the sealing elements (31, 32), **characterised in that** the wall openings (33) are produced as in the form of slits which pass through and extend to the end regions (34, 35) of the tubular body (20) and, in the expanded state, the webs (36) of each sealing element (31, 32) extend substantially at right angles to the longitudinal axis of the implant (30) and form radial loops (37) in the region of each sealing element (31, 32) which are open towards the longitudinal axis of the implant (30), wherein the loops (37) of each sealing element (31, 32) lie substantially in a plane and the loops (37) of the two sealing elements (31, 32) are connected together in the central region (33) by means of webs (36) extending substantially straight and parallel to one another.

2. An implant as claimed in Claim 1, **characterised in that** a plurality of wall openings, (38), which are aligned parallel to one another, are provided.

3. An implant as claimed in one of the preceding claims, **characterised in that** the wall openings (38) extend substantially parallel to the longitudinal axis of the implant (30).

4. An implant as claimed in one of Claims 1 to 2, **characterised in that** the wall openings (38) are arranged at an acute angle to the longitudinal axis of the implant (30).

5. An implant as claimed in one of Claims 1 to 2, **characterised in that** the wall openings (38) extend helically over the periphery of the implant (30).

6. An implant as claimed in one of the preceding claims, **characterised in that** the webs (36) connecting the loops (37) of the two sealing elements (31, 32) in the central region (33) lie approximately on the diameter of a circle (41), which has an increased diameter with respect to the tubular body (20).

7. An implant as claimed in Claim 6, **characterised in that** the diameter of the circle (41) is increased with respect to the diameter of the tubular body (20) by a factor of 2 to 15, preferably 4 to 10.

8. An implant as claimed in one of the preceding claims, **characterised in that** the webs (36) have openings in the region of the loops (37) and/or in the central region (33) for locating a membrane (40).

9. An implant as claimed in one of the preceding claims, **characterised in that** it is convertible from the expanded state into the contracted state by a reversible, elastic or pseudo-elastic deformation.

10. An implant as claimed in one of the preceding claims, **characterised in that** it is made from a shape memory alloy, particularly Nitinol, or from a shape memory polymer.

11. An implant as claimed in one of the preceding claims, **characterised in that** liquid-tight membranes (40) are arranged in or on the sealing elements (31, 32).

12. An implant as claimed in Claim 11, **characterised in that** the membranes (40) are made of ePTFE.

13. An implant as claimed in one of Claims 6 to 12, **characterised in that** the two sealing elements (31, 32) are movable into engagement with the vessel wall defining the vessel opening or shunt connection by compression and/or twisting of the central region (33) and/or the cross-section of the central region (33) is matchable with the cross-section of the vessel opening or of the shunt connection.

14. An implant as claimed in one of the preceding claims, **characterised in that** it is positionable in the body with the aid of a catheter or a spiral coil.

15. An implant as claimed in Claim 14, **characterised in that** the catheter or the spiral coil is fastenable to at least one of the ends (34, 35) of the implant (30).

16. An implant as claimed in Claim 15, **characterised in that** the catheter or the spiral coil may be screwed into one of the tubular ends (34, 35) of the implant or is fastenable by means of a bayonet connector.

## Revendications

1. Implant médical (30) pour l'obturation d'ouvertures de vaisseaux et/ou de liaisons shunt entre des vaisseaux, qui peut être placé de manière invasive au minimum dans un état contracté et qui peut être mis dans un état dilaté, du fait qu'il comprend deux éléments d'étanchéité (31, 32) de diamètre élargi écartés l'un de l'autre, qui étanchéifient l'ouverture du vaisseau ou la liaison shunt des deux côtés, l'implant (30) étant constitué d'un corps tubulaire (20) par la réalisation d'ouvertures de parois (38) avec la formation de nervures (36) au niveau des éléments d'étanchéité (31, 32) présentant un diamètre élargi dans l'état dilaté et dans la zone centrale (33) entre les éléments d'étanchéité (31, 32), **caractérisé en ce que** les ouvertures de parois (38) sont conçues comme des fentes traversantes s'étendant vers les zones d'extrémités (34, 35) du corps tubulaire (20), et dans l'état dilaté, les nervures (36) de chaque élément d'étanchéité (31, 32) s'étendent essentiellement perpendiculairement par rapport à l'axe longitudinal de l'implant (30) et forment, au niveau de chaque élément d'étanchéité (31, 32), des boucles radiales (37) qui sont ouvertes dans l'axe longitudinal de l'implant (30), les boucles (37) de chaque élément d'étanchéité (31, 32) se trouvant essentiellement dans un plan et les boucles (37) des deux éléments d'étanchéité (31, 32) étant reliés entre eux dans la zone centrale (33) par l'intermédiaire de nervures (36) essentiellement droites et parallèles entre elles.

2. Implant selon la revendication 1, **caractérisé en ce que** plusieurs ouvertures de parois (38) parallèles entre elles sont prévues.

3. Implant selon l'une des revendications précédentes, **caractérisé en ce que** les ouvertures de parois (38) s'étendent essentiellement parallèlement à l'axe longitudinal de l'implant (30).

4. Implant selon l'une des revendications 1 ou 2, **caractérisé en ce que** les ouvertures de parois (38) forment un angle aigu avec l'axe longitudinal de l'implant (30).

5. Implant selon l'une des revendications 1 ou 2, **caractérisé en ce que** les ouvertures de parois (38) s'étendent en spirale sur la périphérie de l'implant (30).

6. Implant selon l'une des revendications précédentes, **caractérisé en ce que** les nervures (36) reliant les boucles (37) des deux éléments d'étanchéité (31, 32) dans la zone centrale (33) se trouvent approximativement sur le diamètre d'un cercle (41) qui présente un diamètre élargi par rapport au corps tubulaire (20).

7. Implant selon la revendication 6, **caractérisé en ce que** le diamètre du cercle (41) est élargi par rapport au diamètre du corps tubulaire (20) d'un facteur de 2 à 15, de préférence de 4 à 10.

8. Implant selon l'une des revendications précédentes, **caractérisé en ce que** les nervures (36) présentent, au niveau des boucles (37) et/ou dans la zone centrale (33), des passages pour la fixation d'une membrane (40).

9. Implant selon l'une des revendications précédentes, **caractérisé en ce qu'**il peut passer de l'état dilaté dû à une déformation élastique réversible ou pseudo-élastique à un état contacté.

10. Implant selon l'une des revendications précédentes, **caractérisé en ce qu'**il est constitué d'un alliage à mémoire de forme, plus particulièrement de nitinol, ou d'un polymère à mémoire de forme.

11. Implant selon l'une des revendications précédentes, **caractérisé en ce que**, dans ou sur les éléments d'étanchéité (31, 32), se trouvent des membranes (40) étanches aux liquides.

12. Implant selon la revendication 11, **caractérisé en ce que** les membranes (40) sont constituées de PTFE.

13. Implant selon l'une des revendications 6 à 12, **caractérisé en ce que**, grâce à un aplatissement et/ou une torsion de la zone centrale (33), les deux éléments d'étanchéité (31, 32) peuvent être amenés en appui contre l'ouverture du vaisseau ou la paroi du vaisseau délimitant la liaison shunt et/ou la section transversale de la zone centrale (33) peut être adaptée à la section transversale de l'ouverture du vaisseau ou de la liaison shunt.

14. Implant selon l'une des revendications précédentes, **caractérisé en ce qu'**il peut être positionné dans le corps à l'aide d' un cathéter ou d'un filament spiralé.

15. Implant selon la revendication 14, **caractérisé en ce que** le cathéter ou le filament spiralé peut être fixé au niveau d'au moins une des extrémités (34, 35) de l'implant (30).

16. Implant selon la revendication 15, **caractérisé en ce que** le cathéter ou le filament spiralé peut être vissé dans une des extrémités tubulaires (34, 35) de l'implant ou fixé à l'aide d'une fermeture à baïonnette.
